(19) **Europäisches Patentamt / European Patent Office / Office européen des brevets**

(11) **EP 2 054 367 B1**

(12) **EUROPEAN PATENT SPECIFICATION**

(45) Date of publication and mention
of the grant of the patent:
**22.03.2017 Bulletin 2017/12**

(51) Int Cl.:
**C07C 51/41** (2006.01)     **C07C 51/44** (2006.01)
**C07C 51/46** (2006.01)     **C07C 51/47** (2006.01)
**C07C 57/30** (2006.01)

(21) Application number: **07841187.3**

(22) Date of filing: **22.08.2007**

(86) International application number:
**PCT/US2007/076486**

(87) International publication number:
**WO 2008/024820 (28.02.2008 Gazette 2008/09)**

(54) **PROCESS FOR THE PREPARATION OF SODIUM SALT OF IBUPROFEN OF DIFFERENT PARTICLE SIZES**

VERFAHREN ZUR HERSTELLUNG VON IBUPROFEN-NATRIUMSALZ MIT VERSCHIEDENEN PARTIKELGRÖSSEN

PROCÉDÉ DE PRÉPARATION DE SEL DE SODIUM D'IBUPROFÈNE À DIMENSIONS DE PARTICULES DIFFÉRENTES

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **22.08.2006 US 839338 P**

(43) Date of publication of application:
**06.05.2009 Bulletin 2009/19**

(73) Proprietor: **SI Group, Inc.**
**Schenectady, NY 12309 (US)**

(72) Inventor: **PHAN, Hao, V.**
**Columbia, SC 29209 (US)**

(74) Representative: **Carlisle, Julie**
**Mewburn Ellis LLP**
**City Tower**
**40 Basinghall Street**
**London EC2V 5DE (GB)**

(56) References cited:
**EP-A- 0 813 904     EP-A- 1 410 793**
**WO-A-92/20334**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**BACKGROUND**

Ibuprofen, 2-(4-isobutylphenyl)propionic acid, is a well-known pharmaceutical agent. See for example U.S. Pat. Nos. 3,228,831 and 3,385,886. These patents note that the alkali metal and alkaline earth metal salts of ibuprofen and analogous compounds are water- soluble and valuable for preparation of oral pharmaceutical compositions.

Recently, a need has arisen for providing the sodium salt of ibuprofen (i.e., sodium 2-(4-isobutylphenyl)propionate, or more simply, sodium ibuprofen) in the form of solids on a practical, economical, commercial scale.

EP 0 813 904 describes the preparation of a salt of a pharmaceutically active carboxylic acid compound by reacting the acid in an extruder with less than or equal to one stoichiometric equivalents of a base in melt form.

EP 1 410 793 describes non-effervescent tablets of ibuprofen and the production of these tablets wherein the tablets contain no internal lubricant or disintegrant component.

WO 92/20334 describes use of sodium ibuprofen in pharmaceutical compositions and processes for preparing the *S*-isomer in high enantiomeric purity using a toluene solvent in the manufacturing process.

**BRIEF SUMMARY OF THE INVENTION**

In conducting research for a way to fulfill this need, a process was found which not only achieves that objective, but which additionally enables the production of batches of solid particles of the sodium salt of ibuprofen of different median particle sizes and/or different average (mean) particle sizes depending on how the process is carried out. Moreover, the product recovered from the process after drying is a storage-stable, free flowing particulate product which typically has a water content as determined by Karl Fischer titration in the range of 13 to 14 wt%, and preferably in the range of 13.2 to 13.7 wt%. Thus the product in its various average particle sizes can be easily handled in hoppers, feeding lines, and blending equipment without encountering rat-holing, hang ups, clumping, pluggage, or the like.

One embodiment of this invention enabling achievement of these advantageous features and results is a process which comprises:

A) adding an aqueous sodium hydroxide solution to a non-boiling solution or slurry of ibuprofen in a distillable inert liquid aliphatic hydrocarbon solvent, said solvent having the capability of being distilled along with water at a temperature in the range of 50 to 120°C, the aqueous sodium hydroxide solution being added at a rate that does not cause the resultant reaction mixture to boil before the addition has been completed;

B) after completing the addition in A), removing the water by distilling solvent and water from the reaction mixture until a slurry of sodium ibuprofen in a liquid phase comprised substantially entirely of said solvent is formed;

C) having the temperature of said slurry formed in B) decrease to room temperature, and optionally, having the slurry stand in a quiescent state; and

D) recovering sodium salt of ibuprofen from said slurry by a physical solids- liquid separation procedure;

said process being further characterized by controlling the median particle size of the sodium salt of ibuprofen being formed in the process by selecting and using in A) an aqueous solution of sodium hydroxide with a concentration of 25% to 50 wt% that yields in D), a sodium salt of ibuprofen having a median particle size within a selected range of median particle sizes.

In the above process description letter prefixes A) through D) are used to facilitate reference in a step to another step. The use of such prefixes does not preclude insertion of other suitable steps between consecutively lettered steps.

C) above can be accomplished, for example, by (i) use of refrigeration or other cooling means, (ii) allowing the mixture to stand so that it loses heat to the surroundings, (iii) expediting loss of heat to the surroundings by use of agitation and/or flows of cooling gases, (e.g., fanning under air), or (iv) use of any other way that such cooling can be accomplished, including use of a combination of any two or more cooling procedures.

The "effective concentration" of NaOH as used herein, including in the claims, is defined by the expression:

$$\frac{\text{weight of neat NaOH}}{\text{weight of neat NaOH} + \text{weight of all water charged}}$$

The initial solution or slurry of ibuprofen in a distillable inert liquid aliphatic hydrocarbon solvent (sometimes referred to for convenience as "organic solvent") can contain water before initiating the addition thereto of aqueous sodium hydroxide solution and/or water can be added to the reaction mixture at one or more times apart from the water added by way of the aqueous sodium hydroxide solution. But in any case the amount of the organic solvent relative to the amount of water should be at least enough to distill away substantially all of water present in the reaction mixture and still provide a slurry of sodium ibuprofen in organic solvent. If necessary more organic solvent can be added to the reaction mixture before completing or resuming distillation to remove the water from the reaction mixture.

Typically, recovered sodium salt of ibuprofen is washed at least once with organic solvent, which need not be, but preferably is, the same type of solvent used as the organic solvent in A). After such washing, the sodium salt of ibuprofen is dried.

These and other embodiments and features of this invention will be apparent from the ensuing description and appended claims.

## BRIEF DESCRIPTION OF THE DRAWING

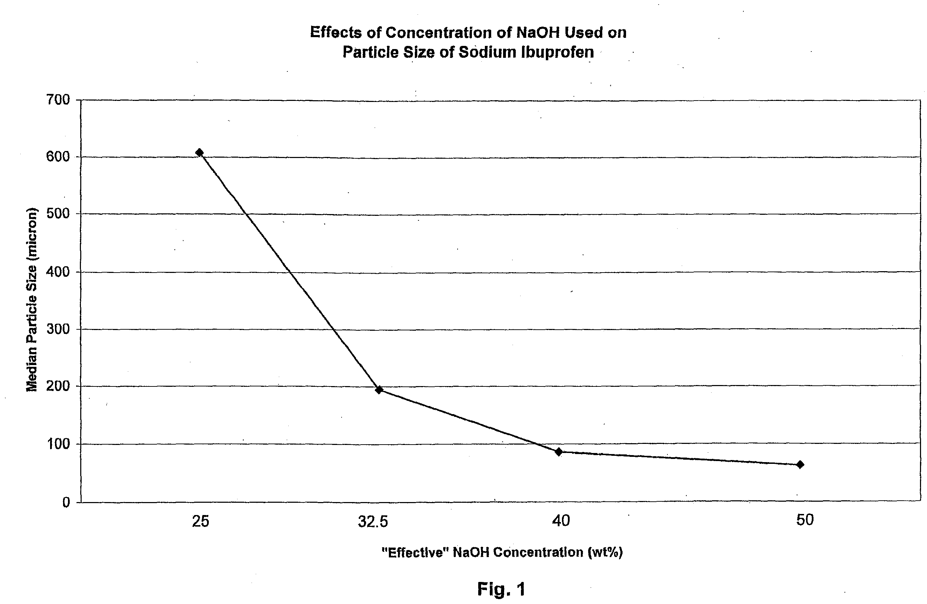

Fig. 1 is a plot showing the effect of variance in NaOH concentration on average particle size of sodium ibuprofen produced experimentally in the practice of this invention.

## FURTHER DETAILED DESCRIPTION OF THE INVENTION

Aqueous Sodium Hydroxide Addition

In A), an effective concentration of sodium hydroxide in the range of 25 to 50 wt% is used in order to achieve any of a wide range of median particle sizes. Note in this connection, Fig. 1. The sodium hydroxide solution can be made using sodium hydroxide and/or sodium oxide.

The total amount of sodium hydroxide used should not exceed the stoichiometric amount needed to completely neutralize the quantity of ibuprofen present in the reaction vessel. Preferably the total amount of sodium hydroxide added is slightly below (e.g., 1-2 mole % below) the stoichiometric amount theoretically required to neutralize all of the ibuprofen present in the reaction mixture. The excess unreacted ibuprofen can be removed by washing the recovered sodium ibuprofen product with organic solvent.

As noted above, the aqueous sodium hydroxide solution is added at a rate that does not cause the mixture to boil before the addition has been completed. However one or more momentary periods of boiling can be tolerated as long as the resultant sodium ibuprofen product formed possesses a desirable targeted median particle size within the range of 50 to 700 microns.

Thus in step A) an aqueous solution of sodium hydroxide is introduced portionwise into a solution of ibuprofen in a distillable liquid aliphatic hydrocarbon solvent The solvent

optionally is in a two-phase liquid mixture with water initially added apart from the water in the aqueous solution of sodium hydroxide. Because the reaction is exothermic, the sodium hydroxide solution is preferably added gradually in increments at a rate that does not result in any boiling occurring until after all of the aqueous solution of sodium hydroxide has been added.

In order to minimize operating costs it is desirable to add the aqueous sodium hydroxide solution to the solution or slurry of ibuprofen in a distillable inert liquid organic solvent at a rate that results in the exothermic reaction producing sufficient heat during the addition period to keep the reaction mixture just below (e.g., two or three degrees below) the boiling temperature of the reaction mixture. Upon completing the addition, sufficient heat energy is then added to the system to boil off the water along with a portion of the organic solvent.

In all cases, the water used in forming the sodium hydroxide solution, should be at least of a purity of potable water. Preferably, distilled water or deionized water is used to avoid the presence of undesirable impurities in the final product. Similarly, the sodium hydroxide used in forming the sodium hydroxide solution should be of high purity and free of toxic or potentially toxic impurities.

Distillable Inert Liquid Organic Solvent

Any aliphatic hydrocarbon solvent that is inert to the reactants and sodium ibuprofen can be used as long as it has the capability of being distilled out of the reaction mixture together with water at a temperature in the range of 50 to 120°C. Such distillation can thus be performed at atmospheric pressure or at reduced pressure. Organic solvents that form with water true azeotropes that boil in this temperature range are preferred but this is not necessary as long as at one or more temperatures in the foregoing temperature range, the organic solvent and water can be concurrently distilled out of a mixture containing them.

Non-limiting examples of aliphatic hydrocarbon solvents that may be selected for use include, n-pentane, n-hexane, cyclohexane, n-heptane and isooctane. Use of n-hexane is recommended.

Because of the uses to which the end product sodium ibuprofen is put, typically pharmaceutical applications, it is preferred to select an organic solvent having the above distillation characteristics that is of high purity to minimize, if not eliminate, contamination of sodium ibuprofen by undesirable impurities from the solvent.

Cooling

Cooling in C) of the slurry formed in B) can be conducted simply by allowing the slurry to stand at room temperature. Alternatively, the slurry may be cooled by use of conventional cooling procedures such as refrigeration, use of vessels equipped with indirect heat exchanger piping or jackets, or the like.Phase Separations

The phase separations used in the process embodiments of this invention are a distillation and a physical solids-liquid separation. Methods of conducting such operations are well known and involve use of readily available equipment and conventional operating procedures. The distillation in B) can be conducted at atmospheric pressure or under reduced pressure conditions. The literature contains considerable information concerning distillation temperatures for binary and ternary mixtures of water and various organic solvents and in any given case such literature should be consulted if the boiling temperature of a proposed water- organic solvent mixture is not already known. If necessary, a trial distillation can be conducted to determine if the proposed water-organic solvent combination will remove the water at a temperature in the range of 50 to 120°C using appropriate reduced pressure conditions if necessary. Experience has shown that n-hexane is an entirely suitable solvent for use in the process.

The physical solids-liquid separation procedure used in D) is typically filtration or centrifugation.

Washing and Drying

Preferably the sodium ibuprofen recovered in D) is washed one or more times with suitably pure organic solvent (preferably a saturated aliphatic hydrocarbon such as n-hexane) to remove excess unreacted ibuprofen as well as other contaminants that may be present. The sodium ibuprofen is then dried typically in an air circulating oven. The drying temperature used should not exceed the temperature at which the product is fully dehydrated. In this connection, the higher the pressure, the higher can be the temperature used for drying. Preferably, when producing sodium ibuprofen dihydrate, the temperature used will be in the range of 1 to 39°C at atmospheric pressure in order to minimize dehydration.

Sodium Ibuprofen Product

In one of its embodiments this invention provides free-flowing particles of sodium 2-(4-isobutylphenyl)propionate having a median particle size in the range of 60 to 610 microns and a water content as determined by Karl Fischer titration in the range of 13 to 14 wt%. In a preferred embodiment these particles also have a mean particle size that is within about 15% of the median particle size of said particles.

In another of its embodiments this invention provides free-flowing particles of sodium 2-(4-isobutylphenyl)propionate having a median particle size in the range of 190 to 610 microns and a water content as determined by Karl Fischer titration in the range of 13 to 14 wt%. In a preferred embodiment these particles also have a mean particle size that is within about 13% of the median particle size of said particles.

In a further embodiment this invention provides free-flowing particles of sodium 2- (4-isobutylphenyl)propionate having a median particle size in the range of 86 to 190 microns and a water content as determined by Karl Fischer titration in the range of 13 to 14 wt%. In a preferred embodiment these particles also have a mean particle size that is within about 13% of the median particle size of said particles.

In still another embodiment of this invention free-flowing particles of sodium 2-(4-isobutylphenyl)propionate are provided having a median particle size in the range of 60 to 86 microns and a water content as determined by Karl Fischer titration in the range of 13 to 14 wt%. In a preferred embodiment these particles also have a mean particle size that is within about 8% of the median particle size of said particles.

In addition to properties and characteristics described hereinabove, the free-flowing particles of sodium ibuprofen produced pursuant to this invention tend to be in the form of platelets when formed using effective concentrations of NaOH in the lower regions of a range of 20 to 60 wt%. On the other hand, when forming the sodium ibuprofen using effective concentrations of NaOH in the upper regions of a range of 20 to 60 wt%, the free-flowing particles of sodium ibuprofen tend to be more needle-like in configuration.

The following Examples illustrate the practice of this invention. They are not intended to limit this invention to only the procedures employed or results obtained in these Examples.

**EXAMPLE 1 - USE OF 25% AQUEOUS NaOH FEED**

With ibuprofen dissolved in hexanes (20.90 wt% ibuprofen) and maintained at 60.5°C, 25 wt% aqueous NaOH (0.98 mol/mol ibuprofen) was fed dropwise over 1.5 hours. The effective concentration of NaOH used was thus 25 wt%. Free water was distilled from the reactor containing two immiscible liquid phases using a Dean-Stark trap until all of the theoretical amount of water was collected. The slurry was then cooled to about room temperature and left stagnant for 60 hrs. The solids were isolated by centrifugation and washed with hexanes. The solids were then tray-dried in a vacuum oven kept at 40°C for 2 hours. Titration for water by the Karl Fischer method yielded a value of 13.50 wt% for the product, sodium 2-(4-isobutylphenyl)propionate. The product was also analyzed for particle size, and these results are given in the Table.

## EXAMPLE 2 - USE OF 50% AQUEOUS NaOH FEED

The experimental procedure of Example 1 was repeated using a 33.3 wt% ibuprofen/hexane solution maintained at 60-62°C with a dropwise feed of 50 wt% aqueous NaOH (0.98 mol/mol ibuprofen), the effective concentration of NaOH used thus being 50 wt%. Free water was distilled from the reactor containing a significant amount of solids until the pot temperature of 62.6°C was reached. The thicker slurry remaining in the reactor was cooled to 23.8°C. The solids were isolated by centrifugation and washed with hexanes. The solids were then tray-dried in a vacuum oven being kept at 40-50°C for 2 hours. Titration for water by the Karl Fischer method yielded a value of 13.26 wt% for the sodium 2-(4-isobutylphenyl)propionate product. The product was also analyzed for particle size, and these results are given in the Table.

## EXAMPLE 3 - USE OF FEED OF WATER FOLLOWED BY 50% AQUEOUS NaOH FEED

The experimental procedure of Example 2 was repeated with ibuprofen dissolved in hexanes (33.3 wt% ibuprofen). The difference between this experimental procedure and that of Example 2 was that water in an amount corresponding to 0.50 gram per gram of neat NaOH to be used, was added to the ibuprofen solution in hexanes before the dropwise feed of the 50 wt% aqueous solution of NaOH. The effective concentration of NaOH used was thus 40 wt%. Free water was distilled from the reactor containing a significant amount of solids until the pot temperature of 62.6°C was reached. Isolation of sodium 2-(4- isobutylphenyl)propionate product was done the same way as in Example 2. The wet product was dried at 40°C. Titration for water by the Karl Fischer method yielded a value of 13.69 wt% for the sodium 2-(4-isobutylphenyl)propionate product. The product was also analyzed for particle size, and these results are given in the Table.

## EXAMPLE 4 - USE OF FEED OF WATER FOLLOWED BY 50% AQUEOUS NaOH FEED

The experimental procedure of Example 3 was repeated with ibuprofen dissolved in hexane (33.3 wt% ibuprofen). The difference between this experimental procedure and that of Example 3 was that more water (1.08 grams per gram of neat NaOH) was added before the dropwise addition of the 50 wt% aqueous solution of NaOH. The effective concentration of NaOH used was thus 32.5 wt%. Free water was distilled from the reactor containing a significant amount of solids until the pot temperature of 62.6°C was reached. Product was isolated as in Example 3. The wet product was dried at 40°C. Titration for water by the Karl Fischer method yielded a value of 13.63 wt% for the product. The product was also analyzed for particle size, and these results are given in the Table.

## EXAMPLE 5 -PREPARATION OF PARTIALLY DEHYDRATED SODIUM SALT OF IBUPROFEN BY OVER-STRIPPING

The experimental procedure of Example 2 was repeated with ibuprofen dissolved in hexane (54.7 wt% ibuprofen). Another difference between this experimental procedure and that of Example 2 was that the final strip temperature was 65.3°C. The wet product was dried at 40°C. Titration for water by the Karl Fischer method yielded a value of 10.44 wt% for the product. The results are given in the Table.

In the following Table, "nd" represents "not determined."

**TABLE**

| Example No. | 1 | 2 | 3 | 4 | 5 |
|---|---|---|---|---|---|
| "Effective" NaOH concentration (wt%) | 25 | 32.5 | 40 | 50 | 50 |
| Final strip temperature, °C | nd | 64.2°C | 62.8°C | 62.6°C | 65.3°C |
| Water, wt% | 13.5 | 13.63 | 13.69 | 13.56 | 10.44 |
| Mean particle size | 676.7 | 217.8 | 92.38 | 67.8 | nd |
| Median particle size | 607 | 194.1 | 86.51 | 63.02 | nd |

(continued)

| Example No. | 1 | 2 | 3 | 4 | 5 |
|---|---|---|---|---|---|
| | | | | | |
| Percentage of particles | Maximum particle size, microns | | | | |
| 10% | 175.2 | 70.75 | 31.71 | 24.95 | nd |
| 15% | 237.1 | 90.94 | 40.96 | 31.69 | nd |
| 50% | 607 | 194.1 | 86.51 | 63.02 | nd |
| 60% | 722.2 | 226.8 | 99.62 | 72.34 | nd |
| 85% | 1143 | 350.2 | 145.3 | 106.2 | nd |
| 90% | 1297 | 396.5 | 160.8 | 117.9 | nd |
| 95% | 1519 | 471.7 | 185.2 | 135.2 | nd |

As can be seen from the data in the Table, as the "effective" concentration of NaOH increases, the particle size decreases. The effects can also be observed graphically in Figure 1, which is a plot of the median particle size versus the "effective" NaOH concentration. As can also be seen from the data in the Table, another advantageous characteristic of the particles of sodium ibuprofen produced pursuant to this invention is the relatively close proximity of the median and mean particle sizes of the particles of the overall mixture.

Components referred to by chemical name or formula anywhere in the specification or claims hereof, whether referred to in the singular or plural, are identified as they exist prior to coming into contact with another substance referred to by chemical name or chemical type (*e.g.*, another component, or a solvent). It matters not what preliminary chemical changes, transformations and/or reactions, if any, take place in the resulting mixture or solution as such changes, transformations, and/or reactions are the natural result of bringing the specified components together under the conditions used according to this disclosure. Thus the components are identified as ingredients to be brought together in performing a desired operation or in forming a desired composition. Also, even though the claims may refer to substances, components and/or ingredients in the present tense ("comprises" or "is"), the reference is to the substance, component or ingredient, as it existed at the time just before it was first contacted, blended or mixed with one or more other substances, components and/or ingredients in accordance with the present disclosure.

This invention is susceptible to considerable variation in its practice. Therefore the above description is not intended to limit, and should not be construed as limiting, the invention to the particular exemplifications given therein. The scope of the invention is as defined in the appended claims.

## Claims

1. A process of producing sodium salt of ibuprofen of controlled median particle size, which process comprises:

A) adding an aqueous sodium hydroxide solution to a non-boiling solution or slurry of ibuprofen in a distillable inert liquid aliphatic hydrocarbon solvent, said solvent having the capability of being distilled along with water at a temperature in the range of 50 to 120°C, the aqueous sodium hydroxide solution being added at a rate that does not cause the resultant reaction mixture to boil before the addition has been completed;
B) after completing the addition in A), removing the water by distilling solvent and water from the reaction mixture until a slurry of sodium ibuprofen in a liquid phase comprised substantially entirely of said solvent is formed;
C) having the temperature of said slurry formed in B) decrease to about room temperature, and optionally, having the slurry stand in a quiescent state; and
D) recovering sodium salt of ibuprofen from said slurry by a physical solids-liquid separation procedure;

said process being further **characterized by** controlling the median particle size of the sodium salt of ibuprofen being formed in the process by selecting and using in A) an aqueous solution of sodium hydroxide with a concentration of 25% to 50 wt% that yields in D), a sodium salt of ibuprofen having a median particle size within a selected range of median particle sizes.

2. A process as in Claim 1 wherein the aqueous sodium hydroxide solution is added to the non-boiling solution or slurry of ibuprofen in A) at a rate such that the heat generated by the exothermic reaction is kept no more than 1°C below the boiling temperature of the reaction mixture to thereby reduce the heat energy input needed to effect the distillation in B).

3. A process as in Claim 1 wherein sodium salt of ibuprofen recovered from the slurry in D) is washed at least once with inert liquid organic solvent and thereafter is dried.

4. A process as in Claim 1 wherein the total amount of sodium hydroxide added in A) is less than the amount required to neutralize the amount of ibuprofen present in A).

5. A process as in Claim 4 wherein sodium salt of ibuprofen recovered from the slurry in D) is washed at least once with inert liquid organic solvent and thereafter is dried.

6. A process as in any of Claims 1-5 wherein the solvent used in A) is a saturated aliphatic hydrocarbon solvent.

7. A process as in any of Claims 1-5 wherein the solvent used in A) is n-hexane.

8. Particles consisting of sodium 2-(4-isobutylphenyl)propionate having a median particle size in the range of 60 to 610 $\mu$m (microns) and a water content as determined by Karl Fischer titration in the range of 13 to 14 wt%.

9. Particles of sodium 2-(4-isobutylphenyl)propionate as in Claim 8 also having a mean particle size that is within 15% of the median particle size of said particles.

10. Particles of sodium 2-(4-isobutylphenyl)propionate as in Claim 8 wherein said median particle size is in the range of 190 to 610 $\mu$m (microns).

11. Particles of sodium 2-(4-isobutylphenyl)propionate as in Claim 10 also having a mean particle size that is within 13% of the median particle size of said particles.

12. Particles of sodium 2-(4-isobutylphenyl)propionate as in Claim 8 wherein said median particle size in the range of 86 to 190 $\mu$m (microns).

13. Particles of sodium 2-(4-isobutylphenyl)propionate as in Claim 12 also having a mean particle size that is within 13% of the median particle size of said particles.

14. Particles of sodium 2-(4-isobutylphenyl)propionate as in Claim 8 wherein said median particle size is in the range of 60 to 86 $\mu$m (microns).

15. Particles of sodium 2-(4-isobutylphenyl)propionate as in Claim 14 also having a mean particle size that is within 8% of the median particle size of said particles.

**Patentansprüche**

1. Verfahren zur Herstellung von Ibuprofen-Natriumsalz mit kontrollierter mittlerer Partikelgröße, wobei das Verfahren Folgendes umfasst:

A) Hinzufügen einer wässrigen Natriumhydroxidlösung zu einer nicht kochenden Lösung oder Suspension von Ibuprofen in einem destillierbaren inerten flüssigen aliphatischen Kohlenwasserstofflösungsmittel, wobei das Lösungsmittel die Fähigkeit aufweist, zusammen mit Wasser bei einer Temperatur im Bereich von 50 bis 120 °C destilliert zu werden, wobei die wässrige Natriumhydroxidlösung in einer Rate hinzugefügt wird, die nicht bewirkt, dass das sich ergebende Reaktionsgemisch kocht, bevor die Hinzufügung abgeschlossen ist;
B) nach dem Abschluss der Hinzufügung in A) Entfernen des Wassers durch Destillieren von Lösungsmittel und Wasser aus dem Reaktionsgemisch, bis eine Suspension von Natrium-Ibuprofen in einer flüssigen Phase, im Wesentlichen vollständig aus dem Lösungsmittel bestehend, gebildet ist;
C) Reduzierenlassen der Temperatur der in B) gebildeten Suspension ungefähr auf Raumtemperatur und optional Stehenlassen der Suspension in einem ruhigen Zustand; und
D) Gewinnen von Ibuprofen-Natriumsalz aus der Suspension durch ein physikalisches Fest-Flüssig-Trennverfahren;

wobei das Verfahren weiter durch das Kontrollieren der mittleren Partikelgröße des in dem Verfahren gebildeten Ibuprofen-Natriumsalzes durch Auswählen und Verwenden einer wässrigen Natriumhydroxidlösung mit einer Konzentration von 25 Gew.-% bis 50 Gew.-% in A), die in D) ein Ibuprofen-Natriumsalz mit einer mittleren Partikelgröße innerhalb einer ausgewählten Spanne an mittleren Partikelgrößen ergibt, gekennzeichnet ist.

2. Verfahren nach Anspruch 1, wobei die wässrige Natriumhydroxidlösung der nicht kochenden Lösung oder Suspension von Ibuprofen in A) in einer solchen Rate hinzugefügt wird, dass die durch die exotherme Reaktion erzeugte Wärme nicht mehr als 1 °C unterhalb der Siedetemperatur des Reaktionsgemischs gehalten wird, um dadurch den Wärmeenergieaufwand zu reduzieren, der benötigt wird, um die Destillation in B) zu bewirken.

3. Verfahren nach Anspruch 1, wobei aus der Suspension in D) gewonnenes Ibuprofen-Natriumsalz mindestens einmal mit inertem flüssigen organischen Lösungsmittel gewaschen wird und danach getrocknet wird.

4. Verfahren nach Anspruch 1, wobei die in A) hinzugefügte Gesamtmenge an Natriumhydroxid geringer als die Menge ist, die erforderlich ist, um die in A) vorhandene Menge an Ibuprofen zu neutralisieren.

5. Verfahren nach Anspruch 4, wobei aus der Suspension in D) gewonnenes Ibuprofen-Natriumsalz mindestens einmal mit inertem flüssigen organischen Lösungsmittel gewaschen wird und danach getrocknet wird.

6. Verfahren nach einem der Ansprüche 1-5, wobei das in A) verwendete Lösungsmittel ein gesättigtes aliphatisches Kohlenwasserstofflösungsmittel ist.

7. Verfahren nach einem der Ansprüche 1-5, wobei das in A) verwendete Lösungsmittel n-Hexan ist.

8. Partikel, bestehend aus Natrium 2-(4-isobutylphenyl)propionat mit einer mittleren Partikelgröße innerhalb der Spanne von 60 bis 610 $\mu$m (Mikrometern) und einem Wassergehalt wie durch Karl-Fischer-Titration bestimmt innerhalb der Spanne von 13 bis 14 Gew.-%.

9. Partikel aus Natrium 2-(4-isobutylphenyl)propionat nach Anspruch 8, auch eine mittlere Partikelgröße aufweisend, die innerhalb von 15 % der mittleren Partikelgröße der Partikel liegt.

10. Partikel aus Natrium 2-(4-isobutylphenyl)propionat nach Anspruch 8, wobei die mittlere Partikelgröße innerhalb der Spanne von 190 bis 610 $\mu$m (Mikrometern) liegt.

11. Partikel aus Natrium 2-(4-isobutylphenyl)propionat nach Anspruch 10, auch eine mittlere Partikelgröße aufweisend, die innerhalb von 13 % der mittleren Partikelgröße der Partikel liegt.

12. Partikel aus Natrium 2-(4-isobutylphenyl)propionat nach Anspruch 8, wobei die mittlere Partikelgröße innerhalb der Spanne von 86 bis 190 $\mu$m (Mikrometern) liegt.

13. Partikel aus Natrium 2-(4-isobutylphenyl)propionat nach Anspruch 12, auch eine mittlere Partikelgröße aufweisend, die innerhalb von 13 % der mittleren Partikelgröße der Partikel liegt.

14. Partikel aus Natrium 2-(4-isobutylphenyl)propionat nach Anspruch 8, wobei die mittlere Partikelgröße innerhalb der Spanne von 60 bis 86 $\mu$m (Mikrometern) liegt.

15. Partikel aus Natrium 2-(4-isobutylphenyl)propionat nach Anspruch 14, auch eine mittlere Partikelgröße aufweisend, die innerhalb von 8 % der mittleren Partikelgröße der Partikel liegt.

**Revendications**

1. Procédé de production de sel de sodium d'ibuprofène à dimension de particules moyenne contrôlée, lequel procédé comprend :

A) le fait d'ajouter une solution aqueuse d'hydroxyde de sodium à une solution sans ébullition ou une suspension d'ibuprofène dans un solvant hydrocarboné aliphatique liquide inerte pouvant être distillé, ledit solvant ayant la capacité d'être distillé avec de l'eau à une température dans la plage allant de 50 à 120 °C, la solution aqueuse d'hydroxyde de sodium étant ajoutée à un taux qui ne fait pas bouillir le mélange réactionnel obtenu avant que l'ajout ait été complété ;

B) après avoir complété l'ajout en A), le fait de retirer l'eau en distillant le solvant et l'eau du mélange réactionnel jusqu'à ce qu'une suspension d'ibuprofène sodique dans une phase liquide composée pratiquement entièrement dudit solvant soit formée ;

C) le fait de faire baisser la température de ladite suspension formée en B) à température ambiante approximativement, et éventuellement, le fait de laisser reposer la suspension ; et

D) le fait de récupérer le sel de sodium d'ibuprofène de ladite suspension par une procédure de séparation solide-liquide physique ;

ledit procédé étant en outre **caractérisé par** le fait de contrôler la dimension des particules moyenne du sel de sodium d'ibuprofène qui sont formées dans le procédé en sélectionnant et en utilisant en A) une solution aqueuse d'hydroxyde de sodium avec une concentration allant de 25 % à 50 % en poids qui produit en D), un sel de sodium d'ibuprofène ayant une dimension de particules moyenne au sein d'une plage sélectionnée de dimensions de particules moyennes.

2. Procédé selon la revendication 1, dans lequel la solution aqueuse d'hydroxyde de sodium est ajoutée à la solution sans ébullition ou la suspension d'ibuprofène en A) à une vitesse telle que la chaleur générée par la réaction exothermique est conservée au maximum à 1 °C en dessous de la température d'ébullition du mélange réactionnel afin de réduire ainsi l'apport d'énergie de chaleur nécessaire pour effectuer la distillation en B).

3. Procédé selon la revendication 1, dans lequel le sel de sodium d'ibuprofène récupéré de la suspension en D) est lavé au moins une fois avec un solvant organique liquide inerte et par la suite est séché.

4. Procédé selon la revendication 1, dans lequel la quantité totale d'hydroxyde de sodium ajoutée en A) est inférieure à la quantité requise pour neutraliser la quantité d'ibuprofène présente en A).

5. Procédé selon la revendication 4, dans lequel le sel de sodium d'ibuprofène récupéré de la suspension en D) est lavé au moins une fois avec un solvant organique liquide inerte et par la suite est séché.

6. Procédé selon l'une quelconque des revendications 1 à 5, dans lequel le solvant utilisé en A) est un solvant hydrocarboné aliphatique saturé.

7. Procédé selon l'une quelconque des revendications 1 à 5, dans lequel le solvant utilisé en A) est le n-hexane.

8. Particules consistant en 2-(4-isobutylphényl)propionate de sodium ayant une dimension de particules moyenne dans la plage allant de 60 à 610 $\mu$m (microns) et une teneur en eau telle que déterminée par le titrage de Karl Fischer dans la plage allant de 13 à 14 % en poids.

9. Particules de 2-(4-isobutylphényl)propionate de sodium selon la revendication 8, ayant également une dimension de particules moyenne qui se situe dans les 15 % de la dimension de particules moyenne desdites particules.

10. Particules de 2-(4-isobutylphényl)propionate de sodium selon la revendication 8, dans lesquelles ladite dimension de particules moyenne se situe dans la plage allant de 190 à 610 $\mu$m (microns).

11. Particules de 2-(4-isobutylphényl)propionate de sodium selon la revendication 10, ayant également une dimension de particules moyenne qui se situe dans les 13 % de la dimension de particules moyenne desdites particules.

12. Particules de 2-(4-isobutylphényl)propionate de sodium selon la revendication 8, dans lesquelles ladite dimension de particules moyenne se situe dans la plage allant de 86 à 190 $\mu$m (microns).

13. Particules de 2-(4-isobutylphényl)propionate de sodium selon la revendication 12, ayant également une dimension de particules moyenne qui se situe dans les 13 % de la dimension de particules moyenne desdites particules.

14. Particules de 2-(4-isobutylphényl)propionate de sodium selon la revendication 8, dans lesquelles ladite dimension de particules moyenne se situe dans la plage allant de 60 à 86 $\mu$m (microns).

**15.** Particules de 2-(4-isobutylphényl)propionate de sodium selon la revendication 14, ayant également une dimension de particules moyenne qui se situe dans les 8 % de la dimension de particules moyenne desdites particules.

Effects of Concentration of NaOH Used on Particle Size of Sodium Ibuprofen

Fig. 1

**EP 2 054 367 B1**

### Patent documents cited in the description

- US 3228831 A
- US 3385886 A
- EP 0813904 A
- EP 1410793 A
- WO 9220334 A